(19) Europäisches Patentamt
European Patent Office
Office européen des brevets

(11) **EP 2 850 211 B1**

(12) **EUROPEAN PATENT SPECIFICATION**

(45) Date of publication and mention
of the grant of the patent:
**08.09.2021  Bulletin 2021/36**

(21) Application number: **13790712.7**

(22) Date of filing: **14.05.2013**

(51) Int Cl.:
*C12Q 1/6851* (2018.01)      *C12Q 1/6869* (2018.01)
*C12Q 1/6809* (2018.01)

(86) International application number:
**PCT/US2013/041031**

(87) International publication number:
**WO 2013/173394 (21.11.2013 Gazette 2013/47)**

(54) **METHOD FOR INCREASING ACCURACY IN QUANTITATIVE DETECTION OF POLYNUCLEOTIDES**

VERFAHREN ZUR VERBESSERUNG DER GENAUIGKEIT BEIM QUANTITATIVEN NACHWEIS VON POLYNUKLEOTIDEN

PROCÉDÉ POUR AUGMENTER LA PRÉCISION DE DÉTECTION QUANTITATIVE DE POLYNUCLÉOTIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **14.05.2012   US 201261646714 P**

(43) Date of publication of application:
**25.03.2015   Bulletin 2015/13**

(73) Proprietor: **iRepertoire, Inc.**
**Huntsville, AL 35806 (US)**

(72) Inventors:
• **WANG, Chunlin**
**Menlo Park**
**California 94025 (US)**
• **HAN, Jian**
**Huntsville**
**Alabama 35802 (US)**

(74) Representative: **Dehns**
**St. Bride's House**
**10 Salisbury Square**
**London EC4Y 8JD (GB)**

(56) References cited:
**WO-A2-2009/152928      US-A1- 2011 081 367**
**US-A1- 2011 294 689**

• Glenn K Fu ET AL: "Counting individual DNA molecules by the stochastic attachment of diverse labels", PNAS, 31 May 2011 (2011-05-31), pages 9026-9031, XP055017200, DOI: 10.1073/pnas.1017621108/-/DCSupplemental Retrieved from the Internet: URL:http://www.pnas.org/content/108/22/9026.full.pdf#page=1&view=FitH [retrieved on 2012-01-23]
• HUBERT HUG ET AL: "Measurement of the number of molecules of a single mRNA species in a complex mRNA preparation.", JOURNAL OF THEORETICAL BIOLOGY, vol. 221, no. 4, 1 April 2003 (2003-04-01) , pages 615-624, XP055017448, ISSN: 0022-5193
• A. M. SMITH ET AL: "Highly-multiplexed barcode sequencing: an efficient method for parallel analysis of pooled samples", NUCLEIC ACIDS RESEARCH, vol. 38, no. 13, 11 May 2010 (2010-05-11), pages e142-e142, XP055017285, ISSN: 0305-1048, DOI: 10.1093/nar/gkq368
• MARGERIDON ET AL.: 'Ultra-deep pyrosequencing of hepatitis B virus quasispecies from nucleoside and nucleotide reverse-transcriptase inhibitor (NRTI)-treated patients and NRTI- naive patients.' J INFECT DIS vol. 199, no. 9, 01 May 2009, pages 1275 - 1285, XP009119714

**(Cont. next page)**

- **WANG ET AL.: 'Characterization of mutation spectra with ultra-deep pyrosequencing: application to HIV-1 drug resistance.' GENOME RES vol. 17, no. 8, August 2007, pages 1195 - 1201, XP002536964**

- **None**

- **None**

## Description

Field of the Invention

The invention relates to methods for quantitative detection of polynucleotides in a mixed sample of polynucleotides. More particularly, the invention relates to methods for increasing accuracy and sensitivity of quantitation of PCR amplification products.

Background of the Invention

Quantitation of DNA, RNA, and gene products is important in a variety of applications-most notably in the areas of microbial and viral detection in clinical samples and in analyzing clinical samples for immunodiversity. Determining the relative numbers of a potentially disease-causing bacteria, for example, could be useful in the clinical setting for providing information regarding patient status, disease progression, likelihood of progression to disease, etc. Quantitation of T cell receptor expression, B cell antibody production, etc., may provide insight into the status of an individual's immune system, the presence or absence of disease, and the progression of change that may be indicative of disease-or even lead to disease.

When evaluating the immune system, researchers are faced with a vast array of diversity and potentially very low copy numbers of targets. Determining the relative amounts of each target (e.g., T cell receptor, B cell antibody) can be a daunting task. Antigen receptors displayed by B cells and T cells have two major parts: B cells have heavy and light chains, and most T cells have $\alpha$ and $\beta$ chains. Estimates are that the human body contains approximately $10^{10}$ lymphocytes, each with a unique combination of gene segments that specify the variable region, the part of the receptor that binds antigen. Each person has an individualized immune repertoire, shaped by three key factors: (1) the genetic polymorphism at the MHC loci; (2) the antigen exposure history; and (3) the constant regulation and modulation of the immune system. Humans are capable of generating $10^{15}$ or more different B and T cells, although not all of these $10^{15}$ B or T cells are present at any given time, due to the history of exposure to various antigens and the process of negative selection during the maturation of immune cells.

Random recombination of heavy-chain segments ($V_H$, $D_H$, and $J_H$) and light-chain segments ($V_K$ and $J_K$ or $V_\lambda$ and $J_\lambda$) produces $V_H D_H J_H$ (heavy chain) and $V_K J_K$ or $V_\lambda J_\lambda$ (light chain) coding units in B cells, and a similar process occurs in T cells. Adding to variable-region diversity is the random deletion of nucleotides at V, D and J segments in the junction position and the random insertion of nucleotides into the regions between the *DJ* and *VD* segments in heavy chain or the regions between the VJ segments in light chain.

One method for quantitating gene expression is to isolate RNA from the samples to be compared, quantitate the RNA by UV spectrophotometry or with a fluorescent dye, and then use equal mass amounts of RNA in real-time RT-PCR. However, RNA quantitation is prone to error from machine or pipette mis-calibration, or dilution, and these methods often require sample dilution for accurate measurement. For samples in which there is already a very low copy number, or at least a relatively low copy number, given the overall numbers of targets, this is very problematic. Furthermore, spectrophotometry cannot be used to detect such small quantities of RNA. It generally takes at least $10^4$ cells to produce enough RNA for accurate quantitation by this method. Using a fluorescent dye can increase sensitivity up to 100-fold, but for many applications even that level of sensitivity is not enough.

Next-generation sequencing technologies have provided opportunities to significantly increase the sensitivity of quantifying DNA and/or RNA targets. Various methods have been developed to improve increasing accuracy of quantification of different polynucleotides in a sample with mixed polynucleotides, including such methods as competitive polymerase chain reaction (PCR), described in U.S. Patent Number 5,213,961 and deep barcode sequencing using unique molecular identifiers (UMI), as described by Smith *et al.* (Smith, A.M., "Quantitative Phenotyping via Deep Barcode Sequencing," Genome Research (2009) 19: 1836-1842).

Unique molecular identifiers, or molecular barcodes, which are also described in Glenn K Fu et al. (PNAS, 31 May 2011, pages 9026-9031) and Hubert Hug et al. (Journal of Theoretical Biology, vo. 221, no. 4, 1 April 2003, pages 615-624) provide an advantage in quantifying copy numbers in a sample. However, if UMI are involved in more than the first round of PCR, the same UMI may be introduced into different targets, resulting in counting errors. Also, the UMI method works based on an ideal, but unrealistic, situation-that is, where both PCR and sequencing technologies are both perfect and no errors are introduced. The UMI strategy operates on the assumption that both PCR and sequencing steps report the underlying targets and UMI fragments free-of-error. However, this is an erroneous assumption because those errors in both PCR and sequencing are inevitable. However, every current sequencing platform is subject to sequencing errors. Two very popular platforms each have error rates of around one percent. When large numbers of sequences are obtained, this sequencing error can create a significant number of artificial targets.

What are needed are methods for improving accuracy of quantification of different polynucleotides in a sample with mixed polynucleotides.

Summary of the Invention

The present invention relates to a method for increasing accuracy and sensitivity of quantitative detection of target polynucleotides in a sample with different polynucleotides, as defined in claim 1. The method may be performed by incorporating into a substantial number of individual target sequences in a pool of target sequences at least one randomly-generated sequence comprising from about 4 to about 15 randomly-generated nucleotides, the at least one randomly-generated sequence forming a unique molecular identifier for an individual target sequence, and a universal adapter sequence (i.e., a primer binding site for a universal primer) to form a target /UMI/adapter polynucleotide; attaching the UMI/universal adapter sequence to the target in a reverse transcription (RT) reaction at 50-60 degree Celsius for RNA targets (A), a primer extension reaction at 50-60 degree Celsius for DNA targets (B); and attaching a second universal adapter to the product of the previous step (A) or (B) by a DNA extension reaction at approximately 70 degree Celsius, and amplifying, with universal primer, products with the universal primer binding site attached at both ends at a temperature of approximately 70 degree Celsius.

In various aspects of the method, the first step of attaching to a target sequence a unique molecular identifier and an adapter sequence is performed by DNA extension or reverse transcription. In various aspects, the first step using DNA extension or reverse transcription is performed at a temperature of from about 50 to about 60 degrees Celsius. In various aspects, the second step of the method is performed at a temperature of from about 65 to about 75 degrees Celsius.

Aspects of the invention involve performing the first step of the method by reverse transcription or DNA extension, using a target-specific primer which comprises a unique molecular identifier sequence of from about 4 to about 15 nucleotides and an adapter sequence.

In various aspects of the invention, the method is performed as an automated method in a closed cassette. The method may also further comprise the steps of sequencing the products produced the amplification step and removing artifacts through statistical filtering. The statistical filtering includes estimating the context-specific error rate based on control DNA sequencing, grouping sequences differing in a single position, assessing the error rate based on the context of the different position, applying a Poisson model to estimate the probability of the sequence with smaller count to be random error and removing those with a probability greater than 0.001 of being random error.

Brief Description of the Drawings

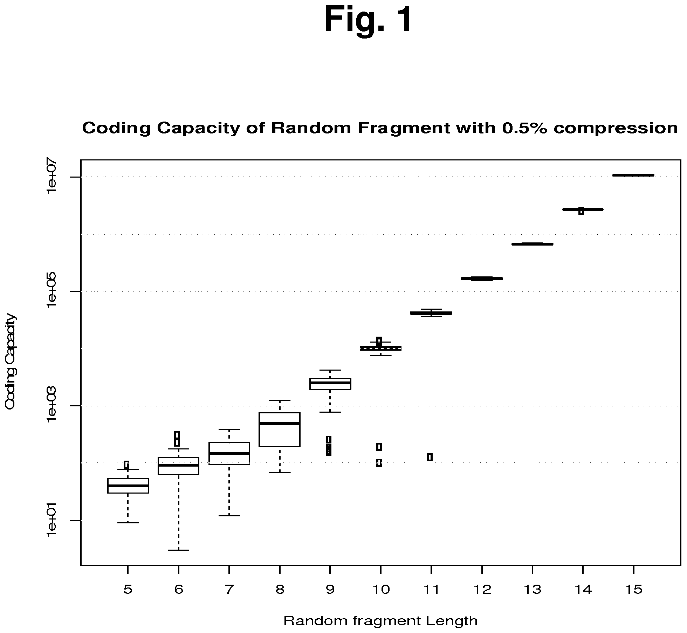

Figure 1 is a plot of the coding capacity of random sequences of various length allowing 0.5% of targets labeled with the same random sequence. The plot is based on data from 10 simulation experiments.

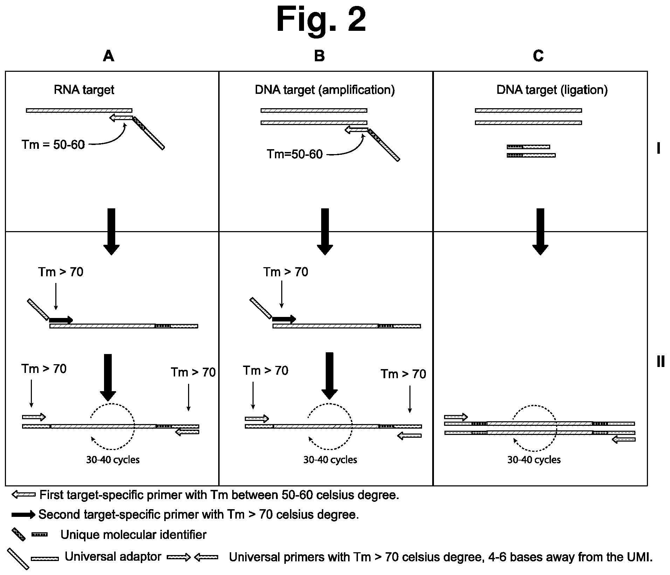

Figure 2 is a diagram of steps to label a target with a unique molecular identifier (UMI), and subsequent amplification steps. For an RNA target (Left panel A), the UMI is introduced by reverse-transcriptase through a reverse-transcription (RT) step where the gene-specific primer is designed with melting temperature (Tm) at between 50 and 60 degrees Celsius. For double-stranded DNA molecules, if specific regions of DNA molecules only are the targets, a UMI (center panel B) is introduced through chain-extension by DNA polymerase with gene-specific primers, which are designed with Tm at between about 50 and about 60 degrees Celsius. After a first step of labeling, a second gene-specific primer and universal primers are added to the reaction with thermostable DNA polymerase. Both the second gene-specific primer and universal primers are designed with Tm greater than 70 degrees Celsius. For pre-selected DNA targets, UMIs are introduced through ligation, where a double adaptor with UMI is ligated to target molecules and UMIs are introduced to a target at both ends. The UMI-labeled targets are then amplified before sequencing.

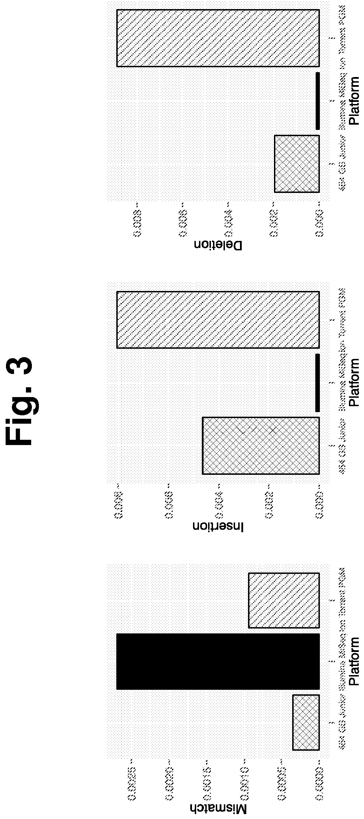

Figure 3 is a context-specific error pattern derived from control DNA sequences determined by the Illumina hiSeq2000 platform. For each row, the height (width) of pattern-filled blocks show the error rate of the last of the triplet changed to either A, C, G or T.

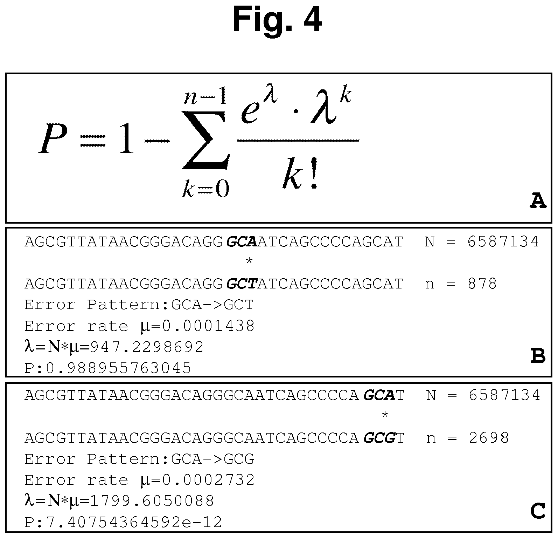

Figure 4 Panel A shows the formula for estimating the odds of whether a minor sequence is generated through artifact, where n is the count of minor sequence in a group, and N the count of major sequence in the same group. A is the expected mean number of sequences identical to the minor sequence, which is computed as $\lambda=N*\mu$, where $\mu$ is the estimated error rate from GCA-GCT in panel B and GCA-GCG in panel C. If the value of P is less than 0.001, it is unlikely that the minor sequence is due to artifacts. Panel B gives an example of a minor sequence with the count 878 being considered as artifact as the value of P is 0.989, which is beyond the 0.001 probability/random error threshold. And panel C gives an example of minor sequences with the count 2698 being considered as authentic as the value of P is 7.4e-12, less than 0.001.

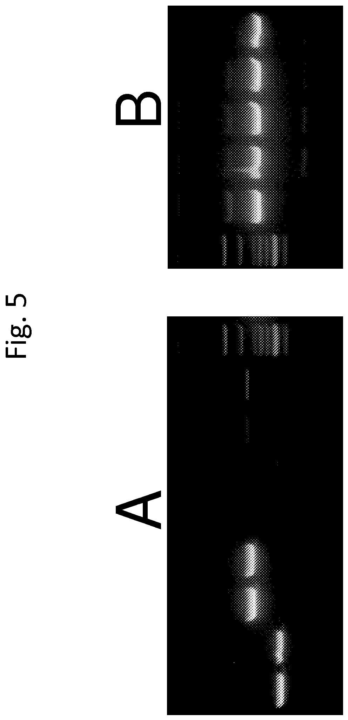

Figures 5A and 5B are photographs of gels containing PCR amplification products produced by the method of the invention. The first four lanes of Figure 4A contain products generated using universal primers and the 2nd four lanes contain products generated using primers for adding a UMI sequence and adapter sequence during RT-PCR, but under the higher temperature conditions of the 2nd/3rd steps of the method. This illustrates that contamination by UMI tagging primers may be avoided using the 3-step method of the invention. The lanes of Figure 4B contain amplification products generated using primers designed for amplification under higher-temperature conditions of the 2nd and 3rd steps of the method.

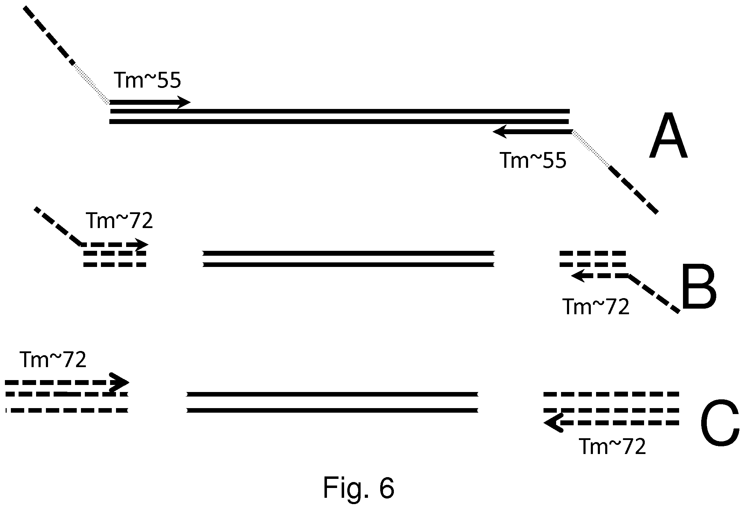

Figure 6 is a drawing illustrating the steps of adding to a target sequence a unique molecular identifier and an adapter sequence (A); performing a first amplification step using at least one forward primer which comprises an adapter sequence and a universal primer binding site sequence (B); and performing a second amplification step using at least one universal primer (C).

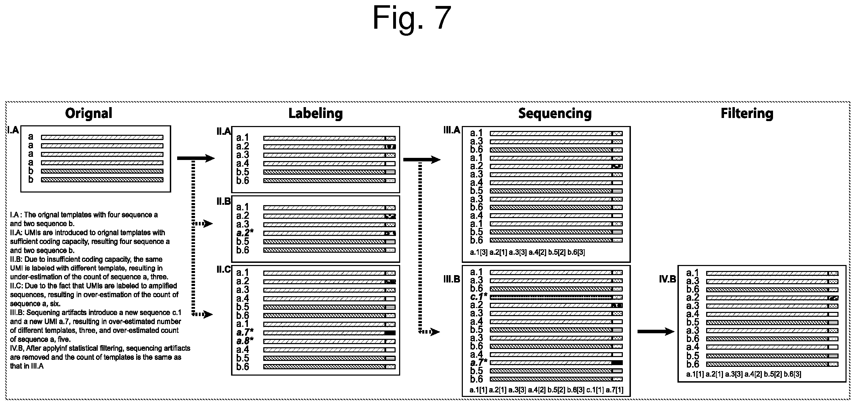

Figure 7 illustrates the benefit of UMI labeling of targets using the method of the invention. Targets in the pool of amplification produced by the present method are sequenced, generally using high-throughput, next-generation sequencing methods. In an ideal situation, each original template (I.A) is labeled with unique UMI (II.A) and sequenced free-of-error (III.A), where the count of the original templates is the same as the count of the combination of target and UMI. If UMIs are too short and with limited coding capacity, the same UMI might be attached to different templates, which will inevitably result in underestimation of the count of the original templates (II.B). If UMIs are attached to targets as they have been amplified, the number of UMIs attached targets is greater than the count of original templates, resulting in over-estimation of the count of certain targets (II.C). If sequencing is not free of error, error could occur in targets, UMI or both. Error occurring in targets results in over-estimation of the count of distinct templates. Error occurring in the UMI region results in over-estimation of the count of certain targets (III.B). With the inventors' statistical filtering technique, those sequencing errors can be detected and removed, which will restore the correct count of distinct targets and the count of each target.

Detailed Description

The inventors have developed a method for increasing the accuracy of detecting the numbers of polynucleotides of substantially the same sequence in a mixed sample of polynucleotides, which may be used in analyses as diverse as those of the immune repertoire, microbiome, gene expression profiling, miRNA profiling, copy number variations, and even prenatal diagnosis of trisomies and drug resistance mutation detections (such as low copy number HIV drug resistance mutation detections).

The invention provides a method for increasing accuracy of quantitative detection of polynucleotides, as defined in claim 1. The method may be performed by incorporating into a substantial number of individual target sequences in a pool of target sequences at least one randomly-generated sequence comprising from about 4 to about 15 randomly-generated nucleotides, the at least one randomly-generated sequence forming a unique molecular identifier for an individual target sequence, and a universal adapter sequence (i.e., a primer binding site for a universal primer) to form a target /UMI/adapter polynucleotide; attaching the UMI/universal adapter sequence to the target in a reverse transcription (RT) reaction at 50-60 degree Celsius for RNA targets (A), a primer extension reaction at 50-60 degree Celsius for DNA targets (B); and attaching a second universal adapter to the product of the previous step (A) or (B) by a DNA extension reaction at approximately 70 degree Celsius, and amplifying, with universal primer, products with the universal primer binding site attached at both ends at a temperature of approximately 70 degree Celsius.

Accurate determination of the composition and quantification of different polynucleotides of varying frequencies in a complex genetic pool is important in a variety of applications-most notably in the areas of microbial and viral detection in clinical samples and in analyzing clinical samples for immunodiversity. Recently, a new method based on deep barcoding or unique molecular identifiers (UMI), as described by Smith et al. (Smith, A.M., "Quantitative Phenotyping via Deep Barcode Sequencing," Genome Research (2009) 19: 1836-1842), has shown promise for decreasing the counting bias introduced during amplification and sequencing. Briefly, each target in a pool is labeled with a unique barcode by covalently attaching a random sequence of a certain length (barcode) to a target polynucleotide before amplification and sequencing. The combination of barcode and target then works as a proxy for the target during amplification and is ultimately sequenced together. At the final step, the unique combination of barcode and target is counted only once. By doing so, the bias introduced during both the amplification stage and the sequencing stage can be suppressed due to the large coding capacity of random sequences of a certain length, which is about $4^N$ (if N is the length of barcode (UMI), for example, the coding capacity of random sequences of the length of 10 is $4^{10} = 1048576$). However, there are three prerequisites for the success of this approach: 1) UMIs have to be long enough to provide sufficient coding capacity so that no two identical targets are labeled with the same UMI; 2) UMIs have to be introduced to target sequences before the amplification steps; and 3) both UMIs and target sequences have to be sequenced without errors. The first requirement can be met by using longer UMIs. The inventors have addressed the second requirement by developing a method that incorporates UMIs in a two-step PCR reaction. The inventors address the third requirement by introducing a new statistical approach to correct for sequencing errors. By combining both methods, they make the UMI strategy more practically useful and increase the accuracy for profiling polynucleotides in a complex genetic pool.

For an RNA target, a UMI is introduced into a target through reverse-transcription (RT) using reverse-transcriptase (Figure 2, left panel A). A gene-specific primer, UMI, and a universal adaptor are synthesized to form one single molecule, where the annealing temperature between the gene-specific primer and a target is designed to be between 50 and 60 Celsius degree. After the RT step, a second gene-specific primer attaching to a second universal adaptor, universal primer is added to reaction, where the annealing temperature between the second gene-specific primer and targets is designed beyond 70 Celsius degree. The second annealing and extension temperature is preferably set to 70 Celsius degree. After this step, a PCR reaction is preferably performed at 95 degrees C for 15 seconds, and 72 degrees for 30-40 cycles.

For DNA targets embedded in large DNA molecules, a UMI is introduced into the target through a regular primer extension step with DNA polymerase (Figure 2, center panel B). A gene-specific primer UMI and a universal adaptor are synthesized in one single molecule, where the annealing temperature between the gene-specific primer and targets is designed between 50 and 60 degrees Celsius. After the primer extension reaction, a second gene-specific primer attaches to a second universal adaptor, and universal primer is added to reaction, the annealing temperature between the second gene-specific primer and targets designed to be above 70 degrees Celsius. The second annealing and extension temperature is preferably set at about 70 degrees Celsius. After this step, a PCR reaction is preferably performed at 95 degrees C for 15 seconds, and 72 degrees C for 30-40 cycles.

The UMI strategy, when used in the absence of the added steps provided by the inventors, operates on the assumption that both PCR and sequencing steps report the underlying target and UMI fragment free of error. However, this is an incorrect assumption because errors in both PCR and sequencing are inevitable.

It is commonly known that the three popular next-generation sequencing platforms on the market today (Illumina HiSeq, Life Technologies Ion Torrent PGM and 454 FLX system) produce sequences with significant numbers of sequencing errors. Figure 3 plots the error pattern of the bench-top version of the three platforms.

For profiling sequences in a complex genetic pool such as 16S rRNA sequencing and immunodiversity studies, the distribution of templates in a sample varies. Sequencing artifacts inevitably distort the result of profiling of nucleic acids in a genetic pool by sequencing. For instance, errors in the UMI region cause an over-estimation of the count of corresponding targets and those errors in the target sequences cause an over-estimation of the number of different targets in the genetic pool. After studying the error patterns of multiple sequencing attempts, several patterns stand out. First, the error rate of any next-generation sequencing platforms is in the range between 0.1% and 5%. Second, errors occur differently in different contexts (i.e., errors are context-specific). Figure 3 shows a context-specific error pattern by the Illumina HiSeq2000 platform.

To suppress artifacts introduced by both PCR and sequencing, the inventors developed a statistical method for identifying those artifacts. This method comprises the steps of 1) estimating error rates by mixing with amplification products of UMI-labeled targets a small amount of control DNA, the sequence of which has been previously determined, sequencing both target and control together, and comparing sequences amplified from control DNA with known sequences, to estimate context-specific pattern of error; 2) organizing target sequences by counting the distribution of unique sequences, where any two unique sequences are grouped if the two sequences differ in a single position; and 3) estimating the odds of the minor sequence in a group of artifacts according to the Poison model (figure 4A).

The inventors noted that if the random label segment is 15 nucleotides in length, it can randomly create about 10756894 unique molecular identifiers to label about 99.5% of around $10^7$ the target polynucleotides.

The term "a target polynucleotide" is used often herein, but it is to be understood that multiple target polynucleotides generally exist within any clinical sample. These may represent sequences derived from, for example, the same or different bacteria, T cells, B cells, viruses, etc. The term, therefore, encompasses labeling of as many single target polynucleotides as can effectively be labeled within a sample. In some cases, such as in the case of immunorepertoire analysis, target polynucleotides may easily number in the millions. Ultimately, UMI-labeled target polynucleotides comprising copies of the same DNA sequences will be individually labeled with different barcodes, each barcode being counted only once to provide a more accurate representation of the numbers of copies of target polynucleotides in a sample. It is therefore important to introduce the UMI label into the method so that it will not be utilized to prime subsequent amplifications and introduce amplification bias into the sample.

The method of the invention may be performed very effectively using a closed cassette and automated methods such as those described in United States Patent Application Publication Number 20100291668A1. The type of quantitation for which the method of the invention is especially useful (i.e., highly diverse targets, low copy numbers in samples) is also especially sensitive to the risk of contamination, which will negatively impact accurate quantitation. The closed system created by the cassette disclosed in United States Patent Application Publication Number 20100291668A1 significantly reduces the risk of contamination, while increasing the efficiency with which many samples may be processed.

When using the automated method described in United States Patent Application Publication Number 20100291668A1, a cassette is insertable into a base machine ("base unit") that operably interfaces with the cassette to provide the necessary movement of a series of parts designed to provide up-and-down vertical movement, horizontal back-and-forth movement, and fluid handling by a cassette pipette which operates within the confines of the area bounded by the top, bottom, ends, and sides of the cassette, these parts being referred to as a cam bar, a lead screw, and a pipette pump assembly, respectively. It is also possible to provide a mechanism that allows the movement of the cassette pipette in any direction in the x-y-z plane, or to allow for circular/rotary movement throughout the enclosed cassette.

At least one of the reagent chambers in the cassette may form a PCR reaction chamber for performing the desired first amplification step (PCR1) and second amplification step (PCR2) of the present invention. Such a reaction chamber may be constructed of different diameter, depth, and wall thickness than other reagent chambers. For example, a reaction chamber preferably will be a thin-walled chamber to aid in thermal conduction between external thermocyclers located in the base unit and the fluid within the reaction chamber. The walls should be tapered so as to easily fit into the thermocycler and make thermal contact with thermocycler without adhering to its surface. The reaction chamber should be of a depth and shape that allows for its fluid volume to be positioned inside the thermocycler. The depth of the PCR chamber should be compatible with the vertical motion of the cassette pipette. Preferably, the chamber will also be accessible to a user's pipette tip if inserted into the chamber through the casette's fill port, and the material used to form the PCR chamber may be optically clear so that the user can see when the pipette tip has reached the bottom of the chamber.

Barcodes, or Unique Molecular Identifiers (UMIs), allow quantitation of PCR products. However, the inventors' experiments with simple addition of UMI sequences in controlled assays in which the number of beginning targets and the relative concentrations of each were known demonstrated that simply adding the UMIs does not give an accurate assessment of the number of targets in, for example, a clinical sample obtained from a human or animal. They hypothesized that utilization of the primers needed for incorporation of the UMI sequences into target-derived polynucleotides could result in additional rounds of amplification in which certain UMIs were added to more than one target. This could result in UMIs representing multiple targets, but being counted as part of a single target, artificially inflating the numbers of some targets. They proposed to develop a method in which tagging/labeling of the target molecules would be performed in a first step, with subsequent steps being designed to limit the influence of the UMI-containing primers so that any primers that remained in the mix would not label additional molecules to an appreciable extent. Counting of products occurs as shown in Fig. 7, where targets may be separated according to their respective sequences and may be quantitated by the numbers of UMIs associated with them in the resulting sequencing results.

The method they designed utilizes primers comprising target-specific sequences for promoting binding to targets to initiate primer extension, as well as randomly-generated UMIs and adapters. The purpose of the adapters is to form a binding site for primers used in next steps, those primers being used to add to resulting polynucleotides nucleotide sequences that form binding sites for universal primers, those primers being chosen for their ability to effectively promote amplification at temperatures of from about 65 to about 75 degrees C. When the primers comprising target-specific sequences are designed for use at lower temperatures, their influence can be limited in the subsequent amplification steps. By using universal primers in the third step (2nd amplification step), amplification bias may be further limited.

Methods for designing primers having desired annealing temperatures are known to those of skill in the art. Methods for generating random nucleotide sequences that may be used as unique molecular identifiers have been described previously and are also known to those of skill in the art.

The present method may also comprise the step of removing a portion of the reaction mix, which contains the products of reverse transcription from the first step of the method, and using that portion for the second amplification reaction. This step may be used to further decrease the influence of the target-specific, UMI-labeled primers in the next two steps.

Sequencing methods, including next-generation high-throughput sequencing methods, are prone to errors, which may be limited to a small percentage-but may produce a significant and unacceptable level of variance when large numbers of nucleotides are sequenced. The method may also further comprise the steps of sequencing the products produced by steps a through c and correcting for sequencing errors using a statistical filtering step using formula I:

$$P \;=\; 1 - \sum_{k=0}^{n-1} \frac{e^{\lambda} \bullet \lambda^{\kappa}}{k!}$$

Particularly when used in the analysis of a human or animal immunorepertoire or the microbial population of, for example, the human intestine, the combination of individually labeling target molecules, semi-quantitatively amplifying those labeled molecules using the two-step amplification of the present invention, using universal primers to decrease amplification bias and improve amplification efficiency, and statistically correcting the sequencing results, will give a much more accurate result and allow a researcher to better determine the types and numbers of immune system cells, antibodies, bacteria, etc. that are present in a given sample.

The invention may be further described by means of the following non-limiting examples.

Examples

The following primers were used to incorporating into each target sequence a unique molecular identifier: milgHC_1: ACACTCTTTCCCTACACGACGCTCTTCCGATCT NNNNNNNNNNNNNTCTGACGTCAGTGGGTAGAT-GGTGGG (SEQ ID NO: 1); milgHC_2:ACACTCTTTCCCTACACGACGCTCTTCCGATCTNNNNNNNNNNNNNTCTG ACTGGATAGACTG ATGGGGGTG (SEQ ID NO: 2); milgHC_3:ACACTCTTTCCCTACAC GACGCTCTT CCGATCTNNNNNNNNNNNNNNTCTGACGTGGATAGACAGATGGGGGT (SEQ ID NO: 3); milgHC_4:ACACTCTT-TCCCTACACGACGCTCTTCCGATCTNNN NNNNNNNNNNTCTG ACAAGGGGTAGAGCTGAGGGTT (SEQ ID NO: 4); milgHC_5: ACACTCTTTCCCTACACGACGCTCTTCCGATCTN NNNNNNNNNNN NTCTGACTGGAT AGAC-CGATGGGGCTG (SEQ ID NO: 5); milgHC_6: ACACTCTTTCCCTACACGAC GCTCTTCCGATCTNNNNNNNNNNNNNNTCTGACGGGGAAGACATTTGGGAAGG (SEQ ID NO: 6); milgHC_7: ACACTCTTTCCCTACACGACGCTCTTCCGATCTNNNNNNNNNN NNNNNTCTGACAGA GGAGGAACATGTCAGGT (SEQ ID NO: 7); and milgHC_8: ACACTCTTTCCCTACACGACGCTCTT CCGATCTNNNNNNNNNNNNNNNNTCTGACG-GG ATAGACAGATGGGGCTG (SEQ ID NO: 8).

TMs of UMI segments targeted for use as annealing sequences were evaluated. Results are listed in Table 1, in order from lowest to highest TM.

**Table 1**

| SEQ ID NO: 7 | milgHC_7 | 51.6 °C | AGAGGAGGAACATGTCAGGT |
|---|---|---|---|
| SEQ ID NO: 6 | milgHC_6 | 52.2 °C | GGGGAAGACATTTGGGAAGG |
| SEQ ID NO: 2 | milgHC_2 | 52.4 °C | TGGATAGACTGATGGGGGTG |
| SEQ ID NO: 3 | milgHC_3 | 52.4 °C | GTGGATAGACAGATGGGGGT |
| SEQ ID NO: 8 | milgHC_8 | 53.5 °C | GGGATAGACAGATGGGGCTG |
| SEQ ID NO: 1 | milgHC_1 | 53.6 °C | GTCAGTGGGTAGATGGTGGG |
| SEQ ID NO: 4 | milgHC_4 | 54.1 °C | AAGGGGTAGAGCTGAGGGTT |
| SEQ ID NO: 5 | milgHC_5 | 55.3 °C | TGGATAGACCGATGGGGCTG |

Templates containing UMIs were generated using reagents as shown in Table 2, under conditions as shown in Table 3.

**Table 2**

| 5x PCR Buffer | 12μl |
|---|---|
| $H_2O$ | 34 μl |
| High fidelity Polymerase | 1 μl |
| Template (1 ng/ug) | μl |
| Amplification primers (10 pmol/ug) | μl |

**Table 3**

| 2-step Cycles | Temp °C | Time |
|---|---|---|
| 1 | 94 | 3 min |
| 30 | 94 | 30 sec |
|  | 72 | 60 sec |
| 1 | 72 | 5 min |

A first primer sequence was synthesized (SEQ ID NO: 9: AATGATACGGCGACCACCGAGAT**CTACACTCTTTCCCTACACGACGCTCTTCC**, with bold print indicating the adapter sequence). A second primer sequence was also synthesized (SEQ ID NO: 10: CAAGCAGAAGACGGCATACGAGATCG **GTCTCGGCATTCCTGCTGAACCGCTCTTCC** (with bold print indicating the adapter sequence).

Illumine primers (SEQ ID NO: 11: AATGATACGGCGACCACCGAGATCTAC ACTCTTTCCCTACACGACGCTCTTCCGATCT and SEQ ID NO: 12: CAAGCAGAAGACGGCATACGAGATCGGTCTCGGCATTCCTGCTGAACCGCTCTTCCG ATCT) served as universal primers.

Primers were tested in both 2-step and 3-step PCR to determine how well they would perform in the method of the invention. Reaction conditions are shown in Tables 4, 5, and 6. Results are shown in Fig. 4A.

**Table 4**

| Reagent | Amount (ul) |
|---|---|
| H2O | 6 |
| Toptaq Master Mix 2x | 12.5 |
| coralload 10x | 2.5 |
| F&R primer mix (10 pmol/ul) | 2 |
| Tamplate (0.0001 pmol/ul) | 2 |
| Total V | 25 |

**Table 5**

| 3-step Cycles | Tem °C | Time |
|---|---|---|
| 1 | 94 | 3 min |
| 30 | 94 | 30 sec |
| | 55 | 30 sec |
| | 72 | 40 sec |
| 1 | 72 | 5 min |

**Table 6**

| 2-step Cycles | Tem °C | Time |
|---|---|---|
| 1 | 94 | 3 min |
| 30 | 94 | 30 sec |
| | 72 | 60 sec |
| 1 | 72 | 5 min |

Universal primers were tested using the following combinations: (1) Sequence ID NO: 12 as forward primer, SEQ ID NO: 11 as reverse primer; (2) Sequence ID NO: 12 as forward primer, UMI primer 1 with SEQ ID NO: 11 as reverse primer; (3) Sequence ID NO: 12 as forward primer, UMI primer 2 with SEQ ID NO: 11 as reverse primer; (4) Sequence ID NO: 12 as forward primer, UMI primer 3 with SEQ ID NO: 11 as reverse primer; and (5) Sequence ID NO: 12 as forward primer, UMI primer 5 with SEQ ID NO: 11 as reverse primer. Results are shown in Fig. 4B.

*Clear Errors Exist in Current Technology*

The inventors began with 4 distinct clones, which were then spiked into a background sample at different concentrations. Following amplification and sequencing, results indicated that there were actually about 50,000 different clones in the sample, a 12,500-fold increase-and a very unacceptable result if the purpose of the work is to quantitate the amount of target DNA in order to evaluate a clinical sample.

*Example of Use of Formula I for Evaluating Results*

For VDJ sequencing, (1-5%) control DNA (e.g., PhiX DNA) was mixed with VDJ amplicons and all were sequenced together. Extract reads for control DNA were based on matches between reads and reference sequence for control DNA. Control DNA sequences were aligned to corresponding reference sequences. The context of specific error patterns were summarized by counting the difference in the alignment between reads and reference (control) DNA, estimating context-specific error rate. For example, if for a small (three nucleotide) fragment GCA, there are 1000 GCA's in all alignments: 991 GCA-> GCA, 3GCA->GCC, 2 GCA->GCG, 2 GCA->GCT, 1 GCA->GC- (deletion) and 1 GCA->GCAx (insertion, x is any one of A, C, G and T), then the error rate for GCA->GCC is 0.003, GCA->GCG is 0.002 and GCA->GCT is 0.002, GCA->GC- is 0.001 and GCA->GCAx is 0.001.

For any two pairs of CDR3's (nucleotide sequences, for example A and B, and frequency(A) > frequency(B)) that are different in a single position (due to either mismatch, insertion or deletion), one can look up to the error rate calculated above according to the context of this difference. Assuming the sequence error is generated through a Poisson distribution, frequency(A) =N and frequency(B) = n, the probability that such B would occur n or more times if it were a sequencing error may be calculated using Formula I.

$$P = 1 - \sum_{k=0}^{n-1} \frac{e^\lambda \cdot \lambda^\kappa}{k!}$$

Formula I

Claims

1. A method for increasing accuracy and sensitivity of quantitative detection of target polynucleotides in a sample with different polynucleotides, the method comprising the steps of

a) using a first target-specific primer to label, by reverse transcription or primer extension at a temperature of from 50 to 60 degrees Celsius, a target polynucleotide with a unique molecular identifier and a universal primer binding site to produce at least one labeled target polynucleotide wherein the nucleotide sequence of the unique molecular identifier is a random-generated sequence comprising 4-15 randomly generated nucleotides;
b) annealing a second target-specific primer attached to a second universal primer binding site to the at least one labeled target polynucleotide at an annealing temperature of above 70 degrees Celsius, and extending the second target-specific primer in an extension reaction at a temperature of 65 to 75 degrees Celsius; and
c) amplifying the at least one labeled target polynucleotide using first and second universal primers to produce multiple copies of the labeled target polynucleotide.

Patentansprüche

1. Verfahren zur Erhöhung der Genauigkeit und Empfindlichkeit des quantitativen Nachweises von Ziel-Polynukleotiden in einer Probe mit verschiedenen Polynukleotiden, wobei das Verfahren die Schritte umfasst des

a) Verwendens eines ersten zielspezifischen Primers, um durch reverse Transkription oder Primer-Verlängerung bei einer Temperatur von 50 bis 60 Grad Celsius ein Ziel-Polynukleotid mit einem eindeutigen molekularen Identifikator und einer universellen Primer-Bindungsstelle zu markieren, um mindestens ein markiertes Ziel-Polynukleotid herzustellen, wobei es sich bei der Nukleotidsequenz des eindeutigen molekularen Identifikators um eine zufällig generierte Sequenz handelt, die 4-15 zufällig generierte Nukleotide umfasst;
b) Anlagerns eines zweiten zielspezifischen Primers, der an eine zweite universelle Primer-Bindungsstelle gebunden ist, an das mindestens eine markierte Ziel-Polynukleotid bei einer Anlagerungstemperatur über 70 Grad Celsius, und Verlängerns des zweiten zielspezifischen Primers in einer Verlängerungsreaktion bei einer Temperatur von 65 bis 75 Grad Celsius; und
c) Amplifizierens des mindestens einen markierten Ziel-Polynukleotids unter Verwendung eines ersten und eines zweiten universellen Primers, um mehrere Kopien des markierten Ziel-Polynukleotids herzustellen.

**Revendications**

1. Procédé pour augmenter la précision et la sensibilité de détection quantitative de polynucléotides cibles dans un échantillon avec différents polynucléotides, le procédé comprenant les étapes consistant à

a) utiliser une première amorce spécifique à une cible pour marquer, par transcription inverse ou extension d'amorce à une température de 50 à 60 degrés Celsius, un polynucléotide cible avec un identifiant moléculaire unique et un site de liaison d'amorce universelle pour produire au moins un polynucléotide cible marqué, dans lequel la séquence nucléotidique de l'identifiant moléculaire unique est une séquence générée de manière aléatoire comprenant 4 à 15 nucléotides générés de manière aléatoire ;
b) recuire une seconde amorce spécifique à une cible fixée à un second site de liaison d'amorce universelle au au moins un polynucléotide cible marqué à une température de recuit supérieure à 70 degrés Celsius, et étendre la seconde amorce spécifique à une cible dans une réaction d'extension à une température de 65 à 75 degrés Celsius ; et
c) amplifier le au moins un polynucléotide cible marqué en utilisant des première et seconde amorces universelles pour produire de multiples copies du polynucléotide cible marqué.

Fig. 1

# Fig. 2

|  | A | B | C |
|---|---|---|---|
|  | RNA target | DNA target (amplification) | DNA target (ligation) |

**I**

Tm = 50-60

Tm=50-60

**II**

Tm > 70

Tm > 70

Tm > 70    Tm > 70

Tm > 70    Tm > 70

30-40 cycles

30-40 cycles

30-40 cycles

⇐══ First target-specific primer with Tm between 50-60 celsius degree.

➡ Second target-specific primer with Tm > 70 celsius degree.

✎ ▬ Unique molecular identifier

╲ ═══ Universal adaptor ══⇒ ⇐══ Universal primers with Tm > 70 celsius degree, 4-6 bases away from the UMI.

EP 2 850 211 B1

13

# Fig. 3

# Fig. 4

$$P = 1 - \sum_{k=0}^{n-1} \frac{e^{\lambda} \cdot \lambda^{k}}{k!}$$

**A**

```
AGCGTTATAACGGGACAGGGCAATCAGCCCCAGCAT    N = 6587134
                        *
AGCGTTATAACGGGACAGGGCTATCAGCCCCAGCAT    n = 878
Error Pattern:GCA->GCT
Error rate μ=0.0001438
λ=N*μ=947.2298692
P:0.988955763045
```

**B**

```
AGCGTTATAACGGGACAGGGCAATCAGCCCCAGCAT    N = 6587134
                                 *
AGCGTTATAACGGGACAGGGCAATCAGCCCCAGCGT    n = 2698
Error Pattern:GCA->GCG
Error rate μ=0.0002732
λ=N*μ=1799.6050088
P:7.40754364592e-12
```

**C**

Fig. 5

Tm~55

Tm~55

A

Tm~72

Tm~72

B

Tm~72

Tm~72

C

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5213961 A

- US 20100291668 A1

**Non-patent literature cited in the description**

- **SMITH, A.M.** Quantitative Phenotyping via Deep Barcode Sequencing. *Genome Research,* 2009, vol. 19, 1836-1842

- **GLENN K FU et al.** *PNAS,* 31 May 2011, 9026-9031
- **HUBERT HUG et al.** *Journal of Theoretical Biology,* 01 April 2003, vol. 221 (4), 615-624